(19) Europäisches Patentamt
European Patent Office
Office européen des brevets

(11) Publication number: **0 372 657 A1**

(12) **EUROPEAN PATENT APPLICATION**

(21) Application number: **89203089.1**

(22) Date of filing: **06.12.89**

(51) Int. Cl.5: **C07D 409/12, C07D 319/18, C07D 319/20, A61K 31/495**

(30) Priority: **08.12.88 NL 8803013**
**07.03.89 NL 8900547**

(43) Date of publication of application:
**13.06.90 Bulletin 90/24**

(84) Designated Contracting States:
**AT BE CH DE ES FR GB GR IT LI LU NL SE**

(71) Applicant: **DUPHAR INTERNATIONAL RESEARCH B.V**
**C.J. van Houtenlaan 36**
**NL-1381 CP Weesp(NL)**

(72) Inventor: **Hartog, Jan**
**C.J. van Houtenlaan 36**
**NL-1381 CP Weesp(NL)**
Inventor: **Mos, Johannes**
**C.J. van Houtenlaan 36**
**NL-1381 CP Weesp(NL)**

(74) Representative: **Muis, Maarten et al**
**OCTROOIBUREAU ZOAN B.V. P.O. Box 140**
**NL-1380 AC Weesp(NL)**

(54) **Anxiolytically active piperazine derivatives.**

(57) The invention relates to a group of new piperazine derivatives having formula 1

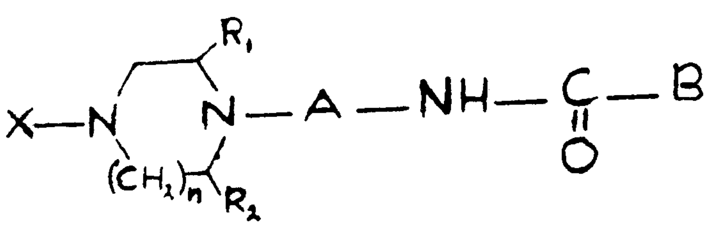

(1)

wherein
- $R_1$ and $R_2$ independently of each other are hydrogen or an alkyl group having 1-3 C atoms,
- n has the value 1 or 2,
- X is an optionally substituted bicyclic group,
- A is a group $-CH_2-CH_2-$, $-CH_2-CH(CH_3)-$ or $-CH(CH_3)-CH_2-$
- B is an aryl group or a heteroaryl group which may be substituted with one or more of the following substituents: halogen, trifluoromethyl, nitrile, alkoxy having 1-3 C-atoms, hydroxy, esterified hydroxy, or alkyl having 1-4 C-atoms, or wherein B is a straight or branched alkyl group having 3-10 C atoms, or a saturated or a partly unsaturated cycloalkyl group having 4-10 carbon atoms or an optionally substituted aralkyl, or an optionally sustituted heteroaralkyl, and salts thereof.
These compounds have psychotropic, and more in particular good anxiolytic properties without the blood pressure being reduced.

EP 0 372 657 A1

## Anxiolytically active piperazine derivatives.

The invention relates to new piperazine derivatives having psychotropic properties.

It has been found that piperazine derivatives of the general formula 1

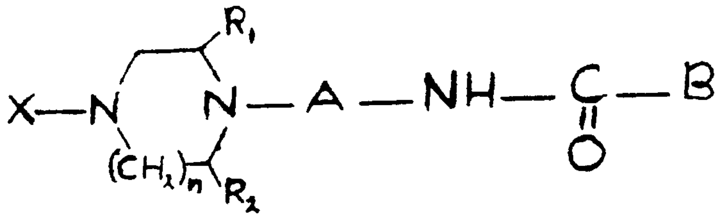

(1)

wherein

$R_1$ and $R_2$ independently of each other are hydrogen or an alkyl group having 1-3 carbon atoms, n has the value 1 or 2, X is one of the groups of the formulas 2-22

2. 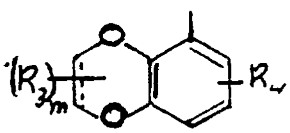 3. 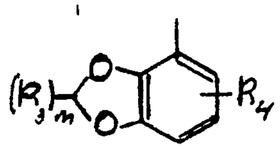 4. 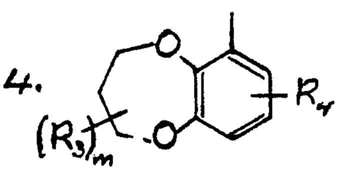

5. 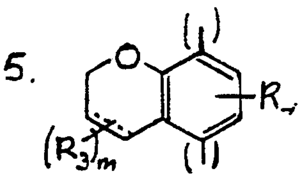 6. 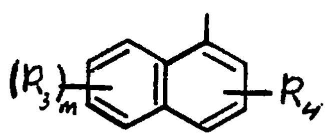 7. 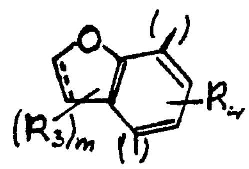

8. 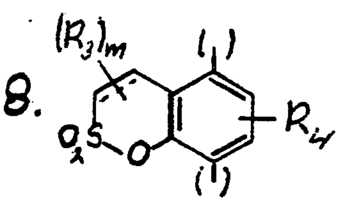 9. 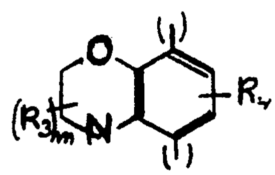 10. 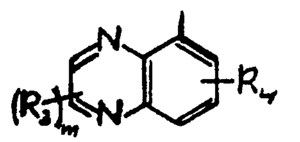

11. 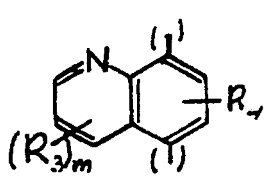 12. 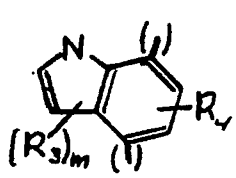 13. 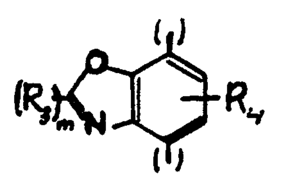

14. 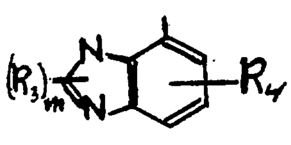 15. 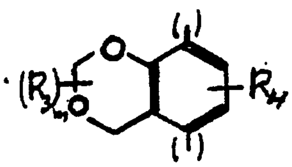 16. 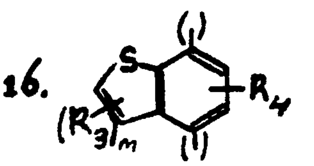

17. 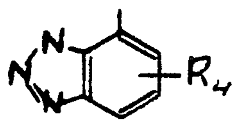 18. 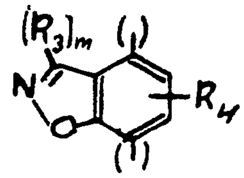 19. 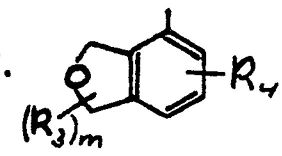

20. 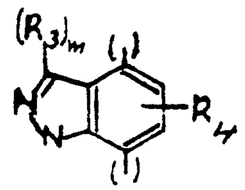 21. 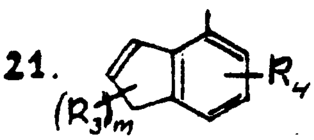 22. 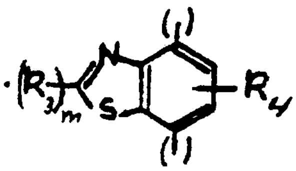

with the proviso that the groups of the formulae 5, 8, 9, 11 and 15 may be bound to the nitrogen atom of the piperazine group via the position 5 or 8, the groups of the formulae 7, 12, 13, 16, 18, 20 and 22 may be bound to the nitrogen atom of the piperazine group via the position 4 or 7, and the groups of the formulae 2, 3, 4, 6, 10, 14, 17, 19 and 21 may be bound to the nitrogen atom of the piperazine group via the indicated positions, and the broken line in formulae 2, 5, 7 and 8 is an optionally present double bond, in which groups $(R_3)_m$ is halogen, straight or branched, saturated or unsaturated alkyl, alkylidene, a straight or branched hydroxyalkyl group which may optionally be esterified or etherified, an alkylcarbonyl group, which may be straight or branched in the alkyl part, an optionally etherified or esterified hydroxy group or an oxo group, an amino group, mono- or dialkylamino group having 1-3 C-atoms per alkyl group, an optionally substituted aminomethyl group, an optionally substituted aryl group or heteroaryl group or an aralkyl group, and m has the value 0, 1 or 2,

**$R_4$** is Cl, Br, CN, hydroxy, a group $-R_5$ or a group $-O-R_5$, $-S-R_5$, $-N-CO-R_5$, $-CO-NH_2$, $-CO-NR_5R_6$, or $-NR_5R_6$ wherein $R_5$ is an alkyl group and $R_6$ is alkyl or hydrogen,

**A** is a group $-CH_2-CH_2-$, $-CH_2-CH(CH_3)-$ or $-CH(CH_3)-CH_2-$,

**B** is an aryl group or a heteroaryl group which may be substituted with one or more of the following substituents: halogen, trifluoromethyl, nitrile, alkoxy having 1-3 C-atoms, hydroxy, esterified hydroxy, or alkyl having 1-4 C-atoms, or wherein B is a straight or branched alkyl group having 3-10 C atoms, or a saturated or a partly unsaturated cycloalkyl group having 4-10 carbon atoms or an optionally substituted aralkyl, or an optionally sustituted heteroaralkyl, have psychotropic and more in particular good anxiolytic properties without the blood pressure being reduced. This is very surprising because structurally closely related compounds are known to have strong blood-pressure lowering properties (EP 0138280 and EP 0185429).

The so-called prodrugs and acid addition salts of the compounds of formula 1 also belong to the present invention. Prodrugs are to be understood to mean herein derivatives from which after administration an active compound of formula 1 is released.

Suitable acids with which the compounds according to the invention can form pharmaceutically acceptable salts are, for example, hydrochloric acid, sulphuric acid, phosphoric acid, nitric acid organic acids, for example, citric acid, fumaric acid, tartaric acid, acetic acid, maleic acid, benzoic acid, p-toluene sulphonic acid, methane sulphonic acid, and the like.

When the compounds according to the invention comprise one or more optically active carbon atoms, both the racemates and the individual enantiomers belong to the present invention.

The anxiolytic activity was established in a number of animal models known and suitable for this purpose.

1) It is known that young rats which are separated from the mother animal and their nestlings produce ultrasonic sounds, the so-called pup vocalisations (see Biochem.Behav. 24, (1986), 1263-1267), These pup vocalisations are characterised by a natural reaction and may be inhibited by means of anxiolytics, in which, in contrast with other models, a removal of a previously caused behaviour inhibition is out of the question.

2) In a second animal model the animal behaviour is used which is recorded after a stimulation of a more or less unpleasant nature has been provided; for example, the natural aversion against light or electric shocks. Such stimuli cause an inhibition of certain behaviour elements and lead to avoiding of the undesired situation. Compounds having an anxiolytic activity remove this inhibition (see Biochem. Behav. 13, (1980), 167-170 and Eur.J.Pharmacol. 4, (1968) 145-151).

3) It is known (see Neuropsychobiology 18, (1987), p. 51) that clinically effective anxiolytics cause an

electroencephalogram (EEG) which is characteristic of these substances.

The compounds having formula 1 are active in the models 1) and 2), and cause EEG's which are very similar to the EEG's caused by clinically active anxiolytics.

The compounds according to the invention are active in dosages which as a rule are between 0.1 and 100 mg/kg after oral administration.

On the basis of the found properties the compounds of formula 1 are suitable for the treatment of those syndromes which are caused by disturbances of the central nervous system, in particular by disturbances of the serotonergic system.

The compounds are suitable, for example, for the treatment of stresses, depressive conditions, sexual behaviour disturbances, memory disturbances, for example, those which occur with senile dementia and the Alzheimer syndrome, migraine, in nausea and vomitting as a result of travelling sickness, and vertigo.

The compounds are suitable in particular for the treatment of various forms of anxiety, for example, compulsive behaviour, obsessions, phobias and panic.

The compounds may be brought into a form suitable for humane application as an anxiolytic in the usual manner, i.e. they may be formulated to compositions suitable for this purpose, preferable for oral administration.

On the basis of their properties, the compounds of formula 1 are to be preferred, wherein n = 1, $R_1$ = $R_2$ = H; A is the ethylene group, B has the meaning mentioned hereinbefore, and X is the group of formula 2, 3, 4, 5, 7 or 8 wherein

$R_3$ is hydrogen, hydroxymethyl, etherified or esterfied hydroxymethyl or oxo, and

$R_4$ is chlorine or methyl in the meta or para position with respect to the piperazine group.

Compounds which are to be preferred in particular are:

(1) 4-fluoro-N-(2-(4-(5-(2-hydroxymethyl-7-chloro-1,4-benzodioxanyl))-1-piperazinyl)ethyl)benzamide;

(2) 4-fluoro-N-(2-(4-(5-(2-hydroxymethyl-7-methyl-1,4-benzodioxanyl))-1-piperazinyl)ethyl)benzamide;

(3) N-(2-(4-(5-(2-hydroxymethyl-7-chloro-1,4-benzodioxanyl))-1-piperazinyl)ethyl)-2-thiophene carboxamide;

(4) 4-fluoro-N-(2-(4-(5-(8-methyl-1,4-benzodioxanyl))-1-piperazinyl)ethyl)-benzamide;

(5) 4-fluoro-N-(2-(4-(5-(7-chloro-1,4-benzodioxanyl))-1-piperazinyl)ethyl)benzamide:

(6) 4-fluoro-N-(2-(4-(5-(7-methyl-1,4-benzodioxanyl))-1-piperazinyl)ethyl)benzamide;

(7) N-(2-(4-(5-(7-chloro-1,4-benzodioxanyl))-1-piperazinyl)ethyl)-2-thiophene carboxamide;

(8) N-(2-(4-(5-(7-methyl-1,4-benzodioxanyl))-1-piperazinyl)ethyl)-2-thiophene carboxamide; and the salts of these compounds.

The new compounds according to the invention may be obtained in manner known for the synthesis of analogous compounds.

The compounds may be obtained, for example, by reaction of a compound of formula 23

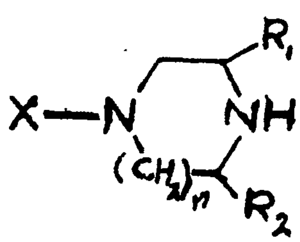

(23)

with a compound of the formula

L - A - NH - CO - B,

in which formulae $R_1$, $R_2$, n, X, A and B have the meanings mentioned hereinbefore and L is a so-called "leaving " group, preferably chlorine, bromine or tosylate.

This reaction may be carried out both with and without an inert organic solvent. Examples of suitable organic solvents are methyl ethyl ketone, dimethyl formamide, tetrahydrofuran, petroleum ether, alcohol and acetonitrile. In order to bind the releasing acid, an acid binder may be used, for example, $NaHCO_3$ or $K_2CO_3$, or an organic base, for example, trietylamine. The reaction temperature usually is between room temperature and the boiling-point of the solvent used.

A modification of this method may be used for the preparation of compounds according to the invention in which A is the ethylene group, by reaction of a compound of formula 23 with a compound of formula 24

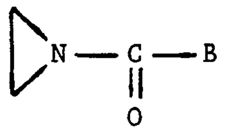

(24)

In these formulae, $R_1$, $R_2$, n, X and B have the above-mentioned meanings.

The reaction components are heated at temperatures between 40-100° C for 1-6 hours preferably without a solvent. However, it is also possible to perform the reaction in an inert solvent, for example, acetone, methyl ethyl ketone or toluene, at a temperature between room temperature and the boiling-point of the solvent used.

The starting compounds of formula 23 to be used for this mode of preparation are described in European Patent Application No. 0189612.

Other modes of preparation suitable for this type of compounds of formula 1 are known from European Patent Applications 0048045, 0138280, 0190472 and from Belgian Patent Specification 892950.

Furthermore, the compounds in which $R_3$ is a hydroxyalkyl group may be obtained by hydrolysis of the corresponding compound in which $R_3$ is an esterified hydroxyalkyl group. Conversely, compounds in which $R_3$ is a hydroxyalkyl group may also be converted to final products in which $R_3$ is an esterified hydroxyalkyl group by esterification in a manner known per se.

Finally, the desired final products of formula 1 in which the symbols have the meanings given hereinbefore, can be obtained in that as the last reaction step one or more protecting groups used for the protection of functional groups, may be removed or converted via methods conventionally used for this purpose.

The individual enantiomers of compounds of formula 1 can be obtained according to methods known per se, for example, by starting from optically active intermediate products, or by resolving an obtained racemate in the enantiomers by means of the techniques conventionally used for this purpose.

The invention will now be described in greater detail, by way of example, with reference to the ensuing specific examples.

## EXAMPLE I

### 4-Fluoro-N-(2-(4-(5-(8-chloro-1,4-benzodioxanyl))-1-piperazinyl)ethyl)benzamide.HCl

9.4 Mmol (2.40 g) of 1-(5-(8-chloro-1,4-benzodioxanyl))piperazine were dissolved in 15 ml of toluene. A solution of 10.5 mmol (1.7 g) of 1-(4-fluorbenzoyl)aziridine in 6 ml of toluene was added in one time. The solution was evaporated to dryness under reduced pressure (15 mm Hg) and the residue was heated at 100° C for 2 hours in an oil bath. The reaction mixture was chromatographed over silica gel with dichloromethane and 1% of methanol as an eluent. The free base obtained in this manner was dissolved in ethanol and treated with 1 equivalent of 0.5.N HCl in ethanol. After addition of ether the title compound was obtained having a melting-point of 174-176° C.

## EXAMPLE II

### N-(2-(5-(7-chloro-1,4-benzodioxanyl))-1-piperazinyl)ethyl)-2-thiophene carboxamide.2HCl

2.18 Mmol of 2-(4-(5-(7-chloro-1,4-benzodioxanyl))-1-piperazinyl)ethylamine were dissolved in 20 ml of dichloromethane and cooled to 0° C. A solution of 2.18 mmol of 2-thiophene carboxylchloride in 10 ml of dichloromethane was added dropwise while stirring. After 30 minutes the reaction was complete according to TLC (5% of MeOH in $CH_2Cl_2$) . The reaction mixture was evaporated under reduced pressure. The residue was chromatographed over silica gel using dichloromethane and 3.5% of methanol as an eluent. The resulting colourless oil was dissolved in ethanol, and then two equivalents of 0.5 N ethanolic HCl were added. After the addition of ether the title compound was obtained having a melting-point of 227-228° C.

EXAMPLE III

4-Fluoro-N-(2-(4-(5-(7-chloro-2-hydroxymethyl-1,4-benzodioxanyl))-1-piperazinyl)benzamide.HCl

4.37 Mmol (2.42 g) of 4-fluoro-N-(2-(4-(5-(7-chloro-2-benzoyloxymethyl-1,4-benzodioxanyl))-1-piperazinyl)ethyl)benzamide were dissolved in 90 ml of ethanol. A solution of 4.64 mmol (2.60 g) of potassium hydroxide in 25 ml of water was added in one time. After stirring at room temperature for 3 hours the reaction mixture was evaporated to dryness under reduced pressure. The resulting residue was dissolved in dichloromethane (75 ml) and washed successively with water (50 ml) and a 5% solution of sodium bicarbonate (50 ml). The organic layer was dried on magnesium sulphate and then evaporated to dryness under reduced pressure. The residue was chromatographed over silica gel using dichloromethane and 3% of methanol as an eluent. 1 Equivalent of alcoholic hydrochloric acid was added to the free base thus obtained, dissolved in 20 ml of ethanol. After dilution with 40 ml of ether the title compound was obtained having a melting-point of 167-168° C.

In an analogous manner the compounds indicated in tables A and B were obtained:

TABLE A

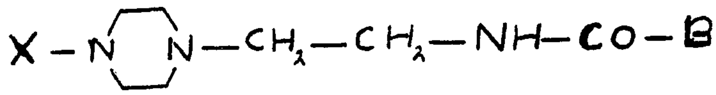

| Comp. no. | method of exemp. | X | B | salt | melting point (C°) |
|---|---|---|---|---|---|
| 1 | I | 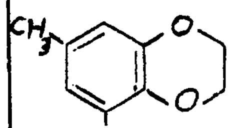 | 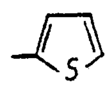 | base | 178-179 |
| 2 | I | 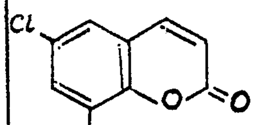 | 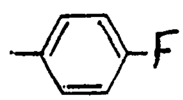 | base | 187-188,5 |
| 3 | I | 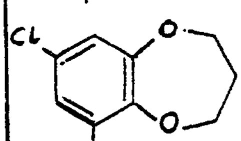 | // | HCl | 204-205 |
| 4 | I | 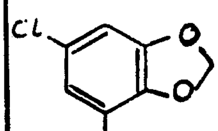 | // | base | 162-163 |
| 5 | I | 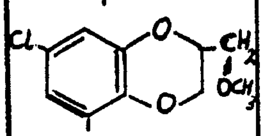 | // | base | 165-167 |
| 6 | I | 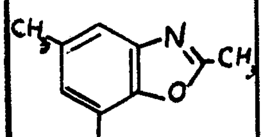 | // | base | 221-222 |

TABLE A

| Comp. no. | method of exemp. | X | B | salt | melting point (C°) |
|---|---|---|---|---|---|
| 7 | I | 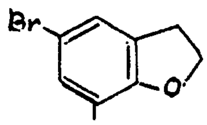 | 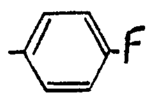 | HCl | 259-263 |
| 8 | I | 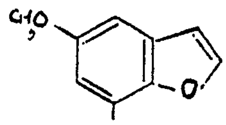 | " | base | 151-152 |
| 9 | I | 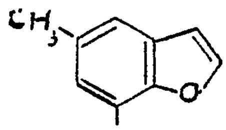 | " | base | 155-156 |
| 10 | I | 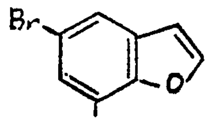 | " | base | 162-163 |
| 11 | I | 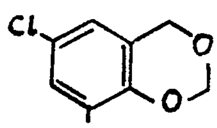 | " | base | 168-171 |
| 12 | I | 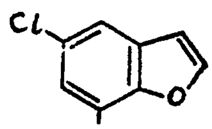 | " | base | 151.5-153.5 |
| 13 | II | 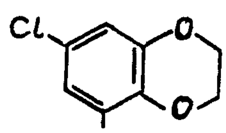 | 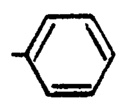 | base | 200-202 |
| 14 | II | " | 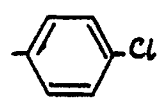 | HCl | 224-226 |

TABLE A

| Comp. no. | method of exemp. | X | B | salt | melting point (°C) |
|---|---|---|---|---|---|
| 15 | II | 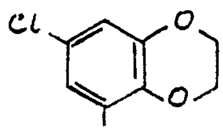 | 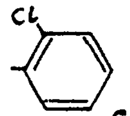 | HCl | 190-192 |
| 16 | II | " | 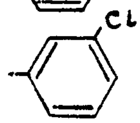 | HCl | 226-228 |
| 17 | II | " | 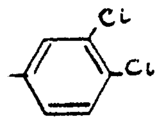 | HCl | 147-148 |
| 18 | II | " | 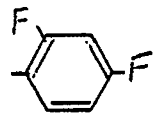 | HCl | 290-293 |
| 19 | II | " | isopropyl. | HCl | 203-205 |
| 20 | II | " | 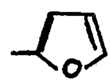 | base | 162-164 |
| 21 | II | " | 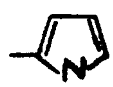 | HCl | 215-217 |
| 22 | I | " | 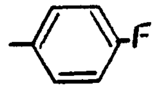 | HCl | 238.5-240 |

| Comp. no. | method of exemp. | X | B | salt | melting point (°C) |
|---|---|---|---|---|---|
| 23 | I | 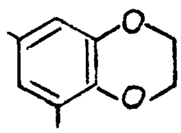 | 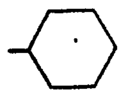 | HCl | 172-174 |
| 24 | II | " | 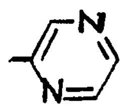 | base | 145-147 |
| 25 | II | " | 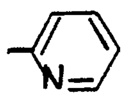 | base | 124-126 |
| 26 | I | 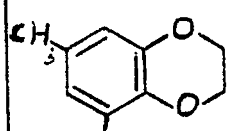 | 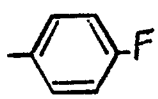 | base | 224-226 |
| 27 | I | 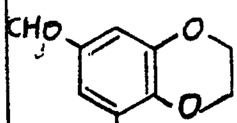 | " | base | 259-261 |
| 28 | II | 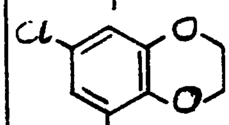 | 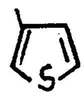 | HCl | 229-231 |
| 29 | I | 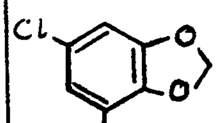 | 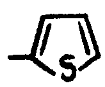 | base | 144-145 |
| 30 | I | 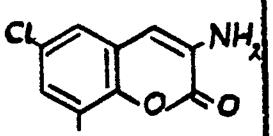 | 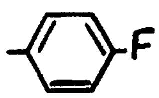 | base | 201-205 (decomp.) |
| 31 | I | 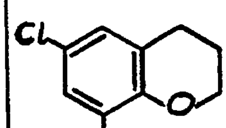 | " | base | 203-206 |

| Comp. no. | method of exemp. | X | B | salt | melting point (°C) |
|---|---|---|---|---|---|
| 32 | I | 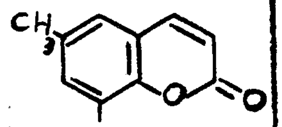 | 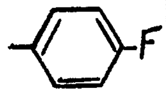 | base | 192-194 |
| 33 | I | 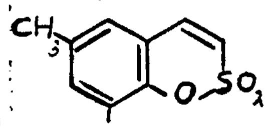 | " | base | 181-183 |
| 34 | I | 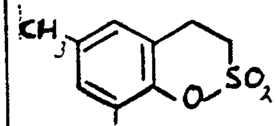 | " | base | 176-178 |
| 35 | I | 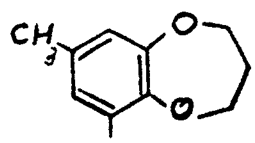 | " | base | 186-189 |
| 36 | I | 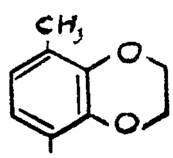 | " | base | 164-166 |
| 37 | I | 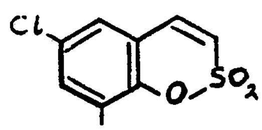 | " | base | 160-162 |
| 38 | I | 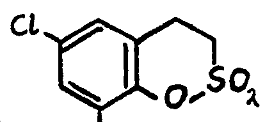 | " | base | 149-152 |
| 39 | I, III | 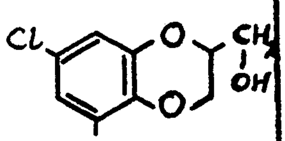 | " | HCl | 167-168 |
| 40 | I, III | " | 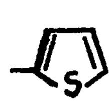 | HCl | 179-195 |

| Comp. no. | method of exemp. | X | B | salt | melting point (°C) |
|---|---|---|---|---|---|
| 41 | I | 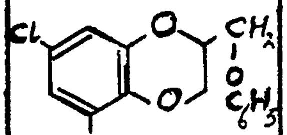 | 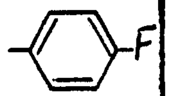 | HCl | 180-183 |
| 42 | III | 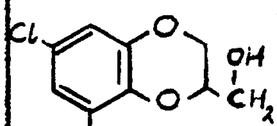 | " | base | 202-204 |
| 43 | III | 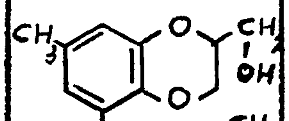 | " | 2 HCl | 232-234 (decomp.) |
| 44 | III | 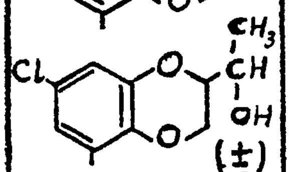 | " | base | 154-157 |
| 45 | III | 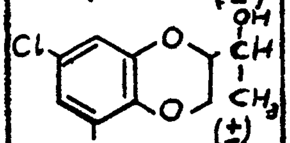 | " | base | 149-151 |
| 46 | I, III | 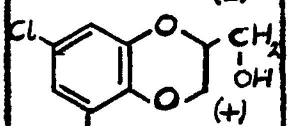 | " | base | $[\alpha]_D^{25}$=+24.4 methanol |
| 47 | I, III | 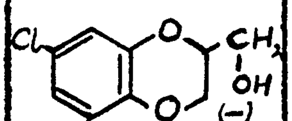 | " | base | $[\alpha]_D^{25}$=-21.3 methanol |
| 48 | I, III | 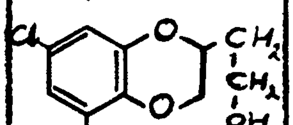 | " | base | 100-103 |
| 49 | I | 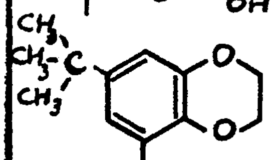 | " | base | 167-169.5 |

| Comp. no. | method of exemp. | X | B | salt | melting point (°C) |
|---|---|---|---|---|---|
| 50 | I | 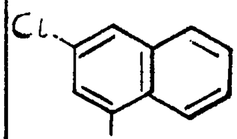 | 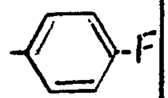 | base | 172-174 |
| 51 | I | 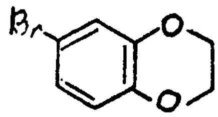 | " | base | 259-261 |

Table B

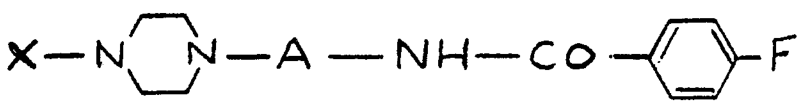

| Comp. no. | method of exemp. | X | A | salt | melting point (°C) |
|---|---|---|---|---|---|
| 52 | I | 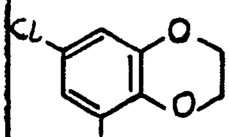 | 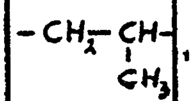 | 2HCl | ± 100 (decomp.) |
| 53 | I | " | 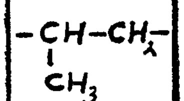 | HCl | 220-223 |
| 54 | I | 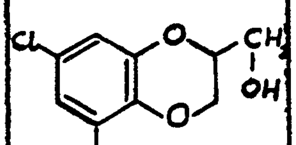 | 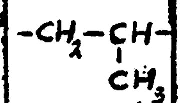 | 2HCl | 162-170 |

## Claims

1. Piperazine derivatives of formula 1

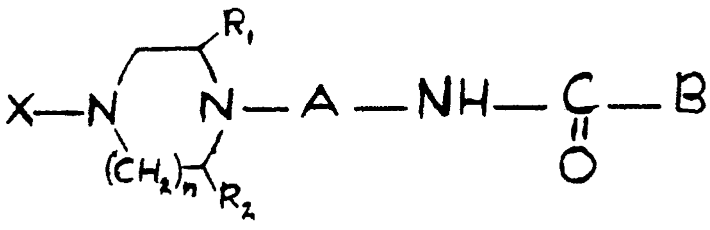

(1)

wherein

$R_1$ and $R_2$ independently of each other are hydrogen or an alkyl group having 1-3 carbon atoms, n has the value 1 or 2, X is one of the groups of the formulas 2-22

2. 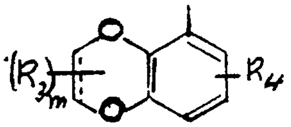 3. 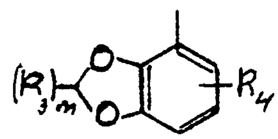 4. 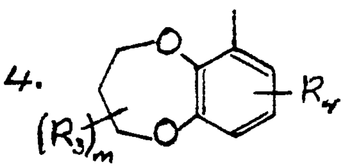

5. 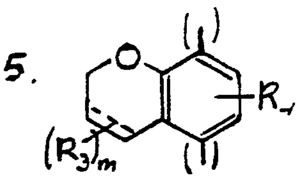 6. 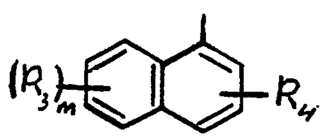 7. 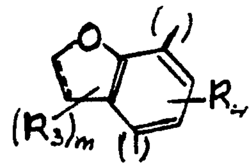

8. 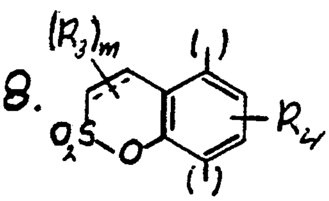 9. 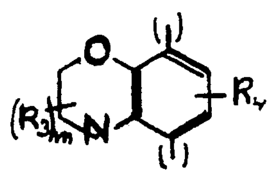 10. 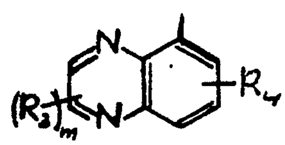

11. 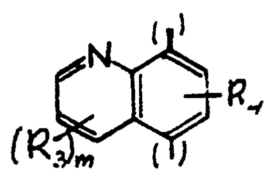 12. 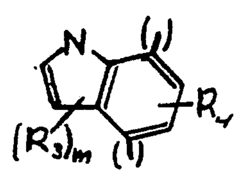 13. 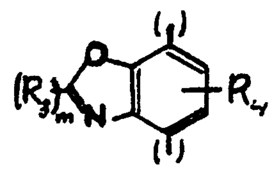

14. 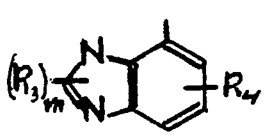 15. 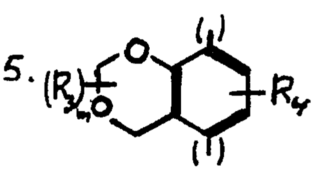 16. 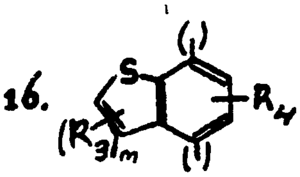

17. 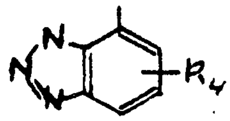 18. 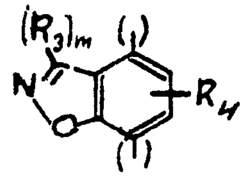 19. 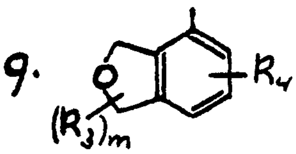

20. 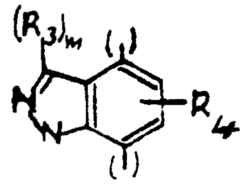 21. 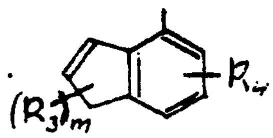 22. 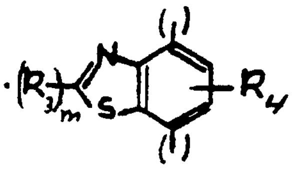

with the proviso that the groups of the formulae 5, 8, 9, 11 and 15 may be bound to the nitrogen atom of the piperazine group via the position 5 or 8, the groups of the formulae 7, 12, 13, 16, 18, 20 and 22 may be bound to the nitrogen atom of the piperazine group via the position 4 or 7, and the groups of the formulae 2, 3, 4, 6, 10, 14, 17, 19 and 21 may be bound to the nitrogen atom of the piperazine group via the indicated positions, and the broken line in formulae 2, 5, 7 and 8 is an optionally present double bond, in which groups $(R_3)_m$ is halogen, straight or branched, saturated or unsaturated alkyl, alkylidene, a straight or branched hydroxyalkyl group which may optionally be esterified or etherified, an alkylcarbonyl group, which may be straight or branched in the alkyl part, an optionally etherified or esterified hydroxy group or an oxo group, an amino group, mono- or dialkylamino group having 1-3 C-atoms per alkyl group, an optionally substituted aminomethyl group, an optionally substituted aryl group or heteroaryl group or an aralkyl group, and m has the value 0, 1 or 2, **$R_4$** is Cl, Br, CN, hydroxy, a group $-R_5$ or a group $-O-R_5$, $-S-R_5$, $-N-CO-R_5$, $-CO-NH_2$, $-CO-NR_5R_6$ or $-NR_5R_6$ wherein $R_5$ is an alkyl group and $R_6$ is alkyl or hydrogen,

**A** is a group $-CH_2-CH_2-$, $-CH_2-CH(CH_3)-$ or $-CH(CH_3)-CH_2-$,

**B** is an aryl group or a heteroaryl group which may be substituted with one or more of the following substituents: halogen, trifluoromethyl, nitrile, alkoxy having 1-3 C-atoms, hydroxy, esterfied hydroxy, or alkyl having 1-4 C-atoms, or wherein B is a straight or branched alkyl group having 3-10 C atoms or a saturated or a partly unsaturated cycloalkyl group having 4-10 carbon atoms or an optionally substituted aralkyl, or an optionally sustituted heteroaralkyl,

prodrugs and pharmacologically acceptable salts thereof.

2. Compounds as claimed in Claim 1, characterised in that $R_1$ and $R_2$ are hydrogen, n has the value 1, A is the ethylene group, B has the meaning given in Claim 1, and X is the group of formula 2, 3, 4, 5, 7 or 8, wherein $R_3$ is hydrogen, hydroxymethyl, etherified or esterified hydroxymethyl or oxo, and $R_4$ is chlorine or methyl in the meta or para position with respect to the piperazine group.

3. Compounds as claimed in Claim 2:

(1) 4-fluoro-N-(2-(4-(5-(2-hydroxymethyl-7-chloro-1,4-benzodioxanyl))-1-piperazinyl)ethyl)benzamide;

(2) 4-fluoro-N-(2-(4-(5-(2-hydroxymethyl-7-methyl-1,4-benzodioxanyl))-1-piperazinyl)ethyl)benzamide;

(3) N-(2-(4-(5-(2-hydroxymethyl-7-chloro-1,4-benzodioxanyl))-1-piperazinyl)ethyl)-2-thiophene carboxamide;

(4) 4-fluoro-N-(2-(4-(5-(8-methyl-1,4-benzodioxanyl))-1-piperazineyl)ethyl)benzamide;

(5) 4-fluoro-N-(2-(4-(5-(7-chloro-1,4-benzodioxanyl))-1-piperazinyl)ethyl)benzamide:

(6) 4-fluoro-N-(2-(4-(5-(7-methyl-1,4-benzodioxanyl))-1-piperazinyl)ethyl)benzamide;

(7) N-(2-(4-(5-(7-chloro-1,4-benzodioxanyl))-1-piperazinyl)ethyl)-2-thiophene carboxamide;

(8) N-(2-(4-(5-(7-methyl-1,4-benzodioxanyl))-1-piperazinyl)ethyl)-2-thiophene carboxamide.

4. Pharmaceutical compositions which comprise a piperazine derivative as an active substance, characterised in that they comprise at least one compound as claimed in Claim 1 an an active substance.

5. A method of preparing anxiolytically active compositions, characterised in that a piperazine derivative as claimed in Claim 1 is brought into a form suitable for administration.

6. A method of preparing piperazine derivatives, characterised in that compounds as claimed in Claim 1 are prepared by reaction of a compound of formula 23

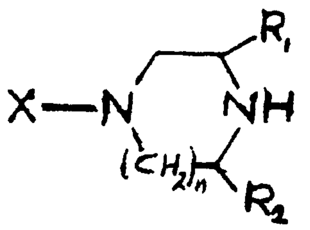

(23)

with a compound of the formula

L - A - NH - CO - B,

in which formulae $R_1$, $R_2$, n, X, A and B have the meanings mentioned in Claim 1.

7. A method as claimed in Claim 6, characterised in that compounds of formula 1 are prepared, wherein A is the ethylene group, by converting a compound of formula 23 with a compound of formula 24

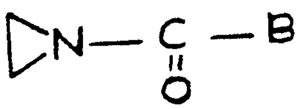

(24)

In which formulae $R_1$, $R_2$, n, X and B have the meanings given in Claim 1.

8. A method as claimed in Claim 6, characterised in that compounds of formula 1 are prepared, wherein $R_3$ is a hydroxyalkyl group, by hydrolysis of the corresponding compound, wherein $R_3$ is an esterified hydroxyalkyl group.

9. A method as claimed in Claim 6, characterised in that compounds of formula 1 are prepared, wherein $R_3$ is an esterified hydroxyalkyl group by esterification of the corresponding compound, wherein $R_3$ is a hydroxyalkyl group.

10. A method as claimed in Claim 6, characterised in that one or more protective groups are removed as the last reaction ste

European Patent Office

# EUROPEAN SEARCH REPORT

Application Number

EP 89 20 3089

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 138 280 (DUPHAR)<br>* Pages 1,2,10; pages 15-18; claims *<br>--- | 1,2,4,6 | C 07 D 409/12<br>C 07 D 319/18<br>C 07 D 319/20<br>A 61 K 31/495 |
| D,A | EP-A-0 185 429 (DUPHAR)<br>* Pages 1-9; claims *<br>----- | 1,2,4,6 | |

TECHNICAL FIELDS SEARCHED (Int. Cl.5)

C 07 D 409/00
C 07 D 403/00
C 07 D 319/00

The present search report has been drawn up for all claims

| Place of search | Date of completion of the search | Examiner |
|---|---|---|
| THE HAGUE | 07-02-1990 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)